Europäisches Patentamt

European Patent Office

Office européen des brevets

(11) Veröffentlichungsnummer: **0 415 055 B1**

(12) **EUROPÄISCHE PATENTSCHRIFT**

(45) Veröffentlichungstag der Patentschrift: **14.12.94**

(21) Anmeldenummer: **90113810.7**

(22) Anmeldetag: **19.07.90**

(51) Int. Cl.5: **C09J 133/06, A61L 15/58**

(54) **Wasserlöslicher druckempfindlicher Hauthaftkleber.**

(30) Priorität: **24.07.89 DE 3924393**

(43) Veröffentlichungstag der Anmeldung:
**06.03.91 Patentblatt 91/10**

(45) Bekanntmachung des Hinweises auf die
Patenterteilung:
**14.12.94 Patentblatt 94/50**

(84) Benannte Vertragsstaaten:
**AT BE CH DE DK ES FR GB IT LI NL SE**

(56) Entgegenhaltungen:
**BE-A- 653 725**
**FR-A- 1 523 183**

(73) Patentinhaber: **RÖHM GMBH**
**Kirschenallee**
**D-64293 Darmstadt (DE)**

(72) Erfinder: **Petereit, Hans-Ulrich**
**Lortzingstrasse 22**
**D-6100 Darmstadt (DE)**
Erfinder: **Roth, Erna**
**Dr.-Kurt-Schuhmacher-Strasse 60, App. 105**
**D-6100 Darmstadt (DE)**

## Beschreibung

Die Erfindung betrifft einen druckempfindlichen Hauthaftkleber zur Befestigung von flächigen flexiblen Substraten, wie Verbänden, Wund- und Heftpflastern oder transdermalen Arzneimitteln, auf der Haut. Von einem druckempfindlichen Kleber spricht man, wenn dieser als getrocknete Schicht dauerhaft weichelastisch ist und beim Aufdrücken eines festen Substrats daran lösbar festklebt. Die getrocknete Kleberschicht braucht jedoch nicht wasserfrei zu sein. Die gewünschte Klebrigkeit tritt manchmal nur dann auf, wenn die Kleberschicht an der Luft abgetrocknet ist und von selbst nicht weiter eintrocknet, jedoch in einem Feuchtigkeitsgleichgewicht mit der Haut steht.

Die Erfindung betrifft weiterhin eine Lösung des Hauthaftklebers und deren Verwendung zum Auftragen auf flexible flächige Substrate. Schließlich betrifft die Erfindung auch Wund- und Heftpflaster und transdermale Arzneimittel.

## Stand der Technik

Gemäß JP-A 76/75 745 (Chem.Abstr.85,99226) verwendet man zum Befestigen von Verbänden auf der Haut eines Patienten ein an sich nicht klebriges Copolymer aus einem Dialkylaminoalkylmethacrylat und einem Alkylmethacrylat zusammen mit einer ausreichenden Menge eines Weichmachers, wie Polypropylenglykol oder Tributylcitrat. Der Kleber ist nicht wasserlöslich. Das gleiche trifft für einen druckempfindlichen Hauthaftkleber gemäß FR 1 523 183 zu, der ein vernetztes Copolymer aus Dimethylaminoäthylmethacrylat und einem Alkylacrylat in Form einer wäßrigen Dispersion enthält.

Aus der US-A 3 321 183 ist auch schon ein druckempfindlicher Hauthaftkleber bekannt, der ein Salz eines Copolymers aus einem aminogruppenhaltigen, äthylenisch ungesättigten, radikalisch polymerisierbaren Monomer und wenigstens einem Alkylester der Acrylsäure enthält. Der Hauthaftkleber ist nicht klar wasserlöslich und wird deshalb in Form einer Lösung in Alkohol, Aceton oder deren Gemisch mit Wasser angewendet, läßt sich aber mit Wasser von der Haut abwaschen. Als salzbildende Säuren werden Salzsäure, organische Sulfonsäuren und Carbonsäuren, wie Essigsäure, Propionsäure und Benzoesäure genannt.

Die EP-A 164 669 beschreibt ein Überzugsmittel für Arzneiformen, das ein Acryl- und/oder Methacrylpolymer mit tertiären Ammoniumgruppen in der Seitenkette in wäßriger Lösung enthält. Die Überzugsmittellösung trocknet zu harten, nicht klebrigen Überzügen auf.

## Aufgabe und Lösung

Der Erfindung liegt die Aufgabe zugrunde, einen druckempfindlichen, dauerelastischen Hauthaftkleber zu schaffen, der ohne Anwendung organischer Lösungsmittel aufgetragen und mit Wasser von der Haut entfernt werden kann.

Der erfindungsgemäße Hauthaftkleber enthält ein Salz eines unvernetzten Copolymers aus einem aminogruppenhaltigen, monoäthylenisch ungesättigten, radikalisch polymerisierbaren Monomer und wenigstens einem Alkylester der Acrylsäure und/oder Methacrylsäure, das in der Salzform wasserlöslich ist. Die Löslichkeit in Wasser wird durch einen hinreichend hohen Anteil des aminogruppenhaltigen Monomeren gewährleistet. Dieser Anteil wird umso größer gewählt, je hydrophober der als Comonomer verwendete Acrylester ist, d.h. je länger sein Alkylrest ist.

Das Copolymer gilt im Sinne der Erfindung als wasserlöslich, wenn es in reinem Wasser bei 20 Grad C eine wenigstens 1-prozentige, klare und homogene Lösung ergibt, in der das Copolymer molekulardispers verteilt vorliegt.

Die dauerelastischen Eigenschaften, welche die Voraussetzung für die Druckempfindlichkeit sind, werden bei einem Feuchtigkeit enthaltenden Film durch eine geeignete Auswahl der Monomerkomponenten des zugrundeliegenden Copolymers oder durch den Zusatz von organischen Weichmachern oder durch eine Kombination dieser Maßnahmen erreicht. Die bevorzugten Hauthaftkleber der Erfindung enthalten 2 bis 200 Gew.-% eines organischen Weichmachers, bezogen auf das Gewicht des Copolymers. Weiterhin können sie mit Hydrophilierungsmitteln, wie Glycerin, Harnstoff oder Sorbitol, versetzt werden, die nur eine begrenzte eigene Weichmacherwirkung haben, aber die Verdunstung des Wassers aus der Kleberschicht verhindern und dadurch die dauerhafte Weichmachung durch den Feuchtigkeitsgehalt gewährleisten. Diese Mittel können in Mengen von z.B. 10 Gew.-% bis zum Mehrfachen des Copolymergewichts angewendet werden.

Wasser hat auf die erfindungsgemäß eingesetzten Copolymeren eine deutlich weichmachende Wirkung, die bei der Anwendung auf der Haut in der Regel zur Wirkung kommt, da die Hauthaftkleberschicht im Feuchtigkeitsgleichgewicht mit der Haut steht. Bei der Herstellung von Hauthaftkleberschichten auf Substraten ist es aus dem gleichen Grund nicht zweckmäßig, die Schacht über diesen Gleichgewichtswert hinaus zu trocknen. Die erwünschte Klebrigkeit tritt in der Regel bei einem Feuchtigkeitsgehalt von 1 bis 10 Gew.-%, bezogen auf das wasserfreie Copolymer, auf.

Der Hauthaftkleber gilt im Sinne der Erfindung als druckempfindlich klebend, wenn er bei dem Klebtest nach European Pharmacopoeia, Second Edition, Part II-7, s. 273 ff (1984) eine Klebkraft von mindestens 1 N/cm aufweist (peel method).

Ausführung der Erfindung

Es ist bekannt, daß die höheren Alkylester der Acryl- und/oder Methacrylsäure den daraus hergestellten Copolymeren Weichheit und Klebrigkeit verleihen. Gleichzeitig machen sie das Copolymer hydrophob und wasserunlöslich. Bei der Festlegung des Anteils des aminogruppenhaltigen, äthylenisch ungesättigten, radikalisch polymerisierbaren Monomers ist daher darauf zu achten, daß er einerseits hoch genug ist, um die Löslichkeit in Wasser zu gewährleisten, andererseits niedrig genug ist, um für einen ausreichenden Anteil des weichmachenden Alkylacrylats oder -methacrylats Raum zu lassen. Wenn die zum Aufbau des Copolymeren verwendeten Monomeren bei keinem Mischungsverhältnis diese beiden Eigenschaften gleichzeitig erreichen lassen, kann ein Weichmacher zuhilfe genommen werden.

Die Klebrigkeit des Copolymers beruht jedoch nicht allein auf seinem Gehalt an Alkylacrylaten oder -methacrylaten, sondern auch auf der Art des aminogruppenhaltigen Monomers. So fördert ein längerer Alkylrest bis zu einem Grenzwert von etwa 10 Kohlenstoffatomen als Bindeglied zwischen der ungesättigten polymerisierbaren Gruppe und der Aminogruppe die Weichheit des Copolymers. Alkylreste oberhalb dieses Grenzwertes mindern die Beweglichkeit der Polymerkette, an die sie gebunden sind, und erhöhen dadurch die Härte. Einen starken Einfluß auf die Weichheit übt das Säureanion aus, als dessen Salz das Copolymer vorliegt. Während die Anionen der Mineralsäuren und der niederen organischen Sulfonsäuren und Carbonsäuren eher die Härte des Copolymeren fördern, wurde überraschend festgestellt, daß sich die Anionen der höheren Carbonsäuren weichmachend auswirken. Dies gilt für Carbonsäuren mit wenigstens vier und besonders mit 8 bis 20 Kohlenstoffatomen. Bevorzugte Carbonsäuren dieser Gruppe sind Caprinsäure, Laurinsäure und Myristinsäure. Wenn mit diesen höheren Carbonsäuren nicht die erforderliche Wasserlöslichkeit erreicht wird, kann man ein Gemisch aus höheren und mittleren Carbonsäuren bzw. Dicarbonsäuren, wie z.B. Adipinsäure, einsetzen. Der Anteil der mittleren Carbonsäuren kann beispielsweise bis zu 30 Mol-% der anionischen Äquivalente ausmachen.

Der Anteil des aminogruppenhaltigen Monomeren an dem Copolymeren beträgt vorzugsweise 30 bis 80 Gew.-%. Bevorzugt sind Ester und Amide der Acryl- und/oder Methacrylsäure, die in der Salzform die Struktur

$$CH_2 = CR - CO - A - Alk - N^+R'_3, X^-$$

haben, worin R ein Wasserstoffatom oder eine Alkylgruppe, vorzugsweise Methyl, A ein Sauerstoffatom oder eine Iminogruppe, vorzugsweise -NH-, Alk eine geradkettige oder verzweigte Alkylengruppe, vorzugsweise mit 2 bis 8 Kohlenstoffatomen, R' gleiche oder verschiedene organische Reste mit bis zu 22 Kohlenstoffatomen, insbesondere Alkyl-, Aryl- oder Aralkylreste, und höchstens zwei der Reste R' Wasserstoffatome sind. $X^-$ stellt das Säureanion dar, für das oben schon Beispiele genannt worden sind. Geeignete aminogruppenhaltige Monomere mit einer tert. Aminogruppe sind Dimethylaminoäthyl-acrylat und -methacrylat, Diäthylaminoäthyl-acrylat und -methacrylat, Dibutylaminoäthyl-acrylat und -methacrylat, Morpholino-äthyl-acrylat und -methacrylat, Piperidino-äthyl-acrylat und -methacrylat, Dimethylamino-2-propyl-acrylat und -methacrylat, Dimethylamino-neopentyl-acrylat und -methacrylat, Dimethylaminoäthyl-acrylamid und -methacrylamid, Diäthylaminoäthyl-acrylamid und -methacrylamid, Dibutylaminoäthyl-acrylamid und -methacrylamid, Morpholino-äthyl-acrylamid und -methacrylamid, Piperidino-äthyl-acrylamid und -methacrylamid, Dimethylamino-2-propyl-acrylamid und -methacrylamid, Dimethylamino-neopentyl-acrylamid und -methacrylamid.

Auch Monomere mit mehreren Aminogruppen, wie z.B. Abkömmlinge des Polyäthylenimins, sind geeignet. Ebenso Monomere, die eine oder mehrere quartäre Ammoniumgruppen tragen. Hierzu gehören N,N-Dimethyl-N-(2-methacryloyloxyäthyl)-aminoessigsäure-betain, N,N-Dimethyl-N-(2-methacryloyl-aminopropyl)-aminoessigsäure-betain, Acryl- und Methacryloxyäthyl-trimethylammonium-chlorid, Acryl - und Methacryloxyäthyl-trimethylammonium-methosulfat, Acryl - und Methacrylamido-äthyl -trimethylammonium-chlorid.

Da quartäre Ammoniumverbindungen mit Carbonsäureanionen schwer zugänglich sind, ist es beim Einsatz der letztgenannten Monomeren schwierig, von der weichmachenden Wirkung höherer Carbonsäureanionen Gebrauch zu machen.

Unter den Alkylestern der Acrylsäure und/oder Methacrylsäure sind diejenigen mit 4 bis 12 Kohlenstoffatomen im Alkylrest bevorzugt, vor allem die Ester der Acrylsäure. Insbesondere n-Butyl-, n-Hexyl-, n-Octyl-, 2-Äthyl-hexyl-, n-Decyl- und n-Dodecylacrylat sind gut geeignet. Die niederen Ester der Acryl- und/oder Methacrylsäure werden in der Regel nur als Comonomere neben den höheren Estern eingesetzt. Daneben können weitere Comonomere am Aufbau des Copolymers beteiligt sein, sofern sie die Wasserlöslichkeit nicht in unzulässiger Weise herabsetzen oder die Härte unzulässig erhöhen. Beispiele sind Acryl- und/oder Methacrylamid, Hydroxyalkylester und Polyalkylenglykolester der Acryl-und/oder Methacrylsäure, Äthylen, Vinylacetat, Vinylpropionat und Vinylpyrrolidon. Zur Herstellung brauchbarer Hauthaftkleber ist die Mitverwendung dieser Monomeren in der Regel nicht erforderlich; ihr Anteil liegt üblicherweise unter 20 Gew.-%.

Das Copolymer kann in der Salzform durch radikalische Copolymerisation der neutralisierten Amino-Monomeren mit den anderen Monomerbestandteilen in wäßriger Lösung erzeugt werden. Es wird jedoch bevorzugt, zunächst das nicht neutralisierte Copolymer herzustellen, indem man anstelle der Monomeren mit einer Ammoniumsalzgruppe das zugrundeliegende Monomer in der Basenform einsetzt. Für die radikalische Polymerisation dieser Monomerengemische stehen verschiedene, seit langem bekannte Polymerisationsverfahren zur Verfügung, z.B. die Polymerisation in Wasser oder in organischen Lösungsmitteln, die Polymerisation in Substanz und, da die Copolymeren in der Basenform weniger wasserlöslich sind, auch die Emulsionspolymerisation in wäßriger Phase, ferner durch "inverse Perlpolymerisation" in organischer Phase". Die organischen Polymerisatlösungen und wäßrigen Polymerisatdispersionen lassen sich z.B. durch Sprühtrocknung in Pulverprodukte überführen. Die Substanzpolymerisate werden im Extruder aufgeschmolzen und zu einem feinen Granulat extrudiert.

Das Molekulargewicht des Copolymers beeinflußt die Viskosität der wäßrigen Lösung des Hauthaftklebers in Abhängigkeit von der Konzentration, bezogen auf den flüssigen Bestandteil. Es liegt vorzugsweise im Bereich zwischen 20.000 und 250.000. Die Viskosität der wäßrigen Lösung des Copolymeren muß durch Wahl einer geeigneten Konzentration dem späteren Verwendungszweck angepaßt werden. Sie soll im allgemeinen nicht mehr als 100 Pa s, vorzugsweise 10 mPa s bis 10 Pa s betragen. Der Polymerisatgehalt der Lösung liegt zweckmäßigerweise etwa im Bereich von 20 bis 70 Gew.-%.

Die wäßrige Lösung kann dadurch hergestellt werden, daß man das feinteilige Copolymer in der Basenform in einer wäßrigen Lösung der Säure rührt, wobei es sich unter Salzbildung auflöst. Die Auflösung kann durch gelindes Erwärmen beschleunigt werden. Der fertigen Lösung können gegebenenfalls Weichmacher und andere Zusätze zugemischt werden. Als Weichmacher eignen sich flüssige, wenigstens begrenzt wasserlösliche organische Stoffe, die mit dem Polymerisat verträglich und unter den Verarbeitungs- und Anwendungsbedingungen nicht oder nicht wesentlich flüchtig sind. Weiterhin sollen sie nicht toxisch sein und nicht bevorzugt in die Haut oder das aufgeklebte Substrat einwandern. Eine ausreichende Verträglichkeit ist gegeben, wenn sich aus dem Copolymeren und dem Weichmacher eine homogene Lösung und aus dieser durch Trocknen ein klarer Film herstellen lassen, oder wenn sich das Copolymer in einem Überschuß des Weichmachers lösen läßt. Als Weichmacher geeignete Stoffe haben in der Regel ein Molekulargewicht zwischen 200 und 20.000 und enthalten eine oder mehrere hydrophile Gruppen im Molekül, z.B. Hydroxy-, Äther- oder Aminogruppen. Beispiele geeigneter Weichmacher sind Citronensäure-triäthylester oder -tributylester, Glycerintriacetat und Polyäthylenglykole 500 bis 20.000.

Anwendung der wäßrigen Hauthaftkleberlösung

Aufgrund ihrer nicht-toxischen und nicht reizenden Eigenschaften kann die wäßrige Hauthaftkleberlösung zur Bildung einer klebenden Oberfläche direkt auf die Haut aufgetragen werden. Nach kurzer Trocknung kann die Klebschicht zur Fixierung von Verbänden, Stützstrümpfen, Korsagen etc. verwendet werden, indem sie auf die Schicht leicht aufgedrückt werden. Das aufgeklebte Substrat kann nach Abschluß der vorgesehenen Einwirkungszeit einfach abgezogen werden, was sich gegebenenfalls durch Anfeuchten mit Wasser erleichtern läßt. Auch bei häufig wiederholten Beschichtungen der gleichen Hautstellen wird keine Reizung beobachtet. Infolge des hydrophilen Charakters der Klebschicht wird die Haut deutlich weniger in Mitleidenschaft gezogen als beim Einsatz konventioneller Kautschukklebschichten. Die auf der Haut verbliebene Klebschicht einschließlich anhaftendem Schmutz, Gewebefasern usw. läßt sich leicht und rasch mit kaltem oder warmem Wasser abwaschen.

Die wäßrige Hauthaftkleberlösung eignet sich in gleicher Weise zur Erzeugung von hauthaftenden druckempfindlichen Klebschichten von Wundpflastern, medizinischen Heftpflastern und Klebebändern oder von transdermal wirkenden, zum Aufbringen auf die Haut vorgesehenen Arzneimitteln. Sie können einen topischen oder systemischen pharmazeutischen Wirkstoff enthalten, der in der Kleberschicht selbst oder in dem beschichteten Substrat enthalten sein kann. Bis zur Anwendung wird die getrocknete Klebschicht, die noch eine ausreichende Feuchtigkeitsmenge enthält, zweckmäßig mit einer Trennfolie geschützt.

Um die Klebschicht zu erzeugen, wird eine 0,01 bis 1 mm dicke Schicht der wäßrigen Hauthaftkleberlösung auf das Substrat aufgebracht und beispielsweise 1 min bis 24 Stunden bei 20 bis 100 Grad C getrocknet, wobei der zur Aufrechterhaltung der gewünschten Klebrigkeit erforderliche Feuchtigkeitsgehalt nicht unterschritten werden darf. Gewünschtenfalls kann die Beschichtung mehrmals wiederholt werden, um eine größere Dicke zu erreichen. Zur Beschichtung kommen flexible flächige Substrate in Betracht, z.B. die bekannten, für Heftpflaster gebräuchlichen Gewebe, Vliese oder Folien. Ebenso können spezielle Polymerschichten, die z.B. als Wirkstoffreservoir dienen, sowie deren Laminate mit Metallfolien mit einer Klebschicht versehen werden. Beim technischen Aufbringen der erfindungsgemäßen Hauthaftkleber wird es als vorteilhaft empfunden, daß keine umweltbelastenden oder explosiven Lösungsmitteldämpfe aus der Trockenzone zu entsorgen sind und daß die Beschichtungsanlage allein mit Wasser gereinigt werden kann.

BEISPIELE

In den Beispielen 1 bis 4 wird ein Copolymer aus 25 Gew.-% Methylmethacrylat, 26 Gew.-% n-Butylmethacrylat und 49 Gew.-% Dimethylaminoäthylmethacrylat eingesetzt.

Beispiel 1

135,2 g des oben genannten Copolymeren werden in 686,7 g Wasser nach Zusatz von 15,9 g Adipinsäure und 78,3 g Laurinsäure ca. 1 Stunde unter Rühren bei ca. 90 Grad C zur Lösung gebracht. Nach dem Abkühlen werden 82,6 g Glycerin und 1,4 g Sorbinsäure zugesetzt. Die Lösung ist dickflüssig und enthält 31,3 % Trockensubstanz. Sie läßt sich durch Rollrakelauftrag mittels einer kontinuierlich arbeitenden Beschichtungsmaschine mit einer Ziehgeschwindigkeit von 0,5 m/min auf eine hautfarbene PVC-Folie (85 $\mu$m dick) auftragen und innerhalb 8 Minuten bei einer Zulufttemperatur von 80 Grad C trocknen.
Die Klebermenge beträgt 0,4 mg/cm² und erreicht eine Klebkraft von 2,5 N/cm. Der Schutz der Klebschicht erfolgt durch eine PVC-Folie. Aus solchen Bahnen herausgeschnittene Pflasterverbände halten mehrere Tage auf der Haut und verursachen keine Reizung.

Beispiel 2

113 g des oben genannten Copolymeren werden in 720 g Wasser nach Zusatz von 13 g Adipinsäure und 54 g Laurinsäure unter Rühren über ca. 1 Stunde durch Erwärmen auf 90 Grad C gelöst. Nach dem Abkühlen werden 100 g Glycerin zugesetzt.
Diese Lösung ist klar und enthält 28 % Trockensubstanz. Sie besitzt eine Viskosität von 750 mPas und trocknet auf der Haut zu klebenden Schichten mit ca. 1,3 N/cm Klebkraft, die Verbände und Stützstrümpfe sicher fixieren. Nach deren Entfernen lassen sich Klebstoffreste von der Haut einfach mit Wasser abwaschen.

Beispiel 3

118 g des oben genannten Copolymeren werden unter Rühren mit 14 g Adipinsäure und 68 g Laurinsäure in 800 g Wasser durch Erwärmen über ca. 1 Stunde in Lösung gebracht.
Die klare Lösung mit einem Trockengehalt von 20 % ist dünnflüssig. Sie kann auf Aluminiumfolie aufgetragen werden und erreicht nach dem Trocknen eine Klebkraft von ca. 5,7 N/cm.

Beispiel 4

175 g des oben genannten Copolymeren werden unter Rühren mit 19 g Glutarsäure und 84 g Laurinsäure in 649 g Wasser durch Erwärmen gelöst. Nach dem Abkühlen werden 73 g Glycerin zugesetzt. Diese Lösung mit 35,1 % Trockensubstanz trocknet nach dem Rakelauftrag auf Aluminiumfolie zu Schichten mit ca. 4,9 N/cm Klebkraft.

Beispiel 5

200 g einer wäßrigen Dispersion mit einem Gehalt von 30 Gew.-% eines Polymeren aus 35 Teilen Acrylsäureethylester, 25 Teilen Methacrylsäuremethylester, 30 Teilen 3-Dimethylamino-2,2-dimethylpropyl-1-methacrylat und 10 Teilen eines Gemisches aus $C_{12}$- und $C_{14}$-Alkyl-methacrylat werden auf 60 Grad C erwärmt und mit 33,2 g einer 10 %igen wäßrigen Lösung von Adipinsäure gemischt. Anschließend erwärmt man auf 90 Grad C, fügt 16,3 g Laurinsäure hinzu und rührt den Ansatz bei dieser Temperatur über 2 Stunden. Die abgekühlte Lösung ist dünnflüssig und enthält 31,9 % Trockensubstanz und bildet nach dem Verdunsten des Lösungsmittels klebrige Schichten.

**Patentansprüche**

1.  Dauerelastischer, druckempfindlicher Hauthaftkleber, enthaltend ein Salz eines unvernetzten Copolymers aus einem aminogruppenhaltigen, monoäthylenisch ungesättigten, radikalisch polymerisierbaren Monomer und wenigstens einem Alkylester der Acryl- und/oder Methacrylsäure,

    dadurch gekennzeichnet,
    daß das Copolymer das Salz wenigstens einer höheren organischen Carbonsäure mit 8 bis 20 Kohlenstoffatomen oder eines Gemisches einer solchen höheren Carbonsäure mit bis zu 30 Mol-% (der anionischen Äquivalente) an mittleren Carbonsäuren ist und einen Anteil des aminogruppenhaltigen Monomers im Bereich von 30 bis 80 Gew.-% (bezogen auf das Gewicht des Copolymers) enthält und in der Salzform in Wasser löslich ist.

2. Hauthaftkleber nach Anspruch 1, dadurch gekennzeichnet, daß das Copolymer einen Alkylester der Acryl-und/oder Methacrylsäure mit 4 bis 14 Kohlenstoffatomen im Alkylrest enthält.

3. Hauthaftkleber nach Anspruch 1 oder 2, dadurch gekennzeichnet, daß er 2 bis 200 Gew.-% eines Weichmachers für das Salz des Copolymers enthält.

4. Hauthaftkleber nach einem oder mehreren der Ansprüche 1 bis 3, dadurch gekennzeichnet, daß er 1 bis 10 Gew.-% Wasser, bezogen auf das wasserfreie Copolymer, enthält.

5. Wäßrige Lösung, enthaltend einen Hauthaftkleber nach einem oder mehreren der Ansprüche 1 bis 4 und 20 bis 90 Gew.-% Wasser.

6. Verwendung der wäßrigen Lösung des Hauthaftklebers nach Anspruch 5 zum Auftragen auf ein flächiges flexibles Substrat und anschließende Trocknung.

7. Wundpflaster, enthaltend einen Hauthaftkleber nach einem oder mehreren der Ansprüche 1 bis 4.

8. Transdermal wirkendes, an der Haut haftendes Arzneimittel, enthaltend einen topischen oder systemischen pharmazeutischen Wirkstoff und einen Hauthaftkleber nach einem oder mehreren der Ansprüche 1 bis 4.

## Claims

1. A permanently elastic, pressure-sensitive skin adhesive containing a salt of a copolymer which is not cross-linked and consists of an amino group-containing, monoethylenically unsaturated, radically polymerisable monomer and at least one alkylester of acrylic and/or methacrylic acid, characterised in that the copolymer is the salt of at least one higher organic carboxylic acid having 8 to 20 carbon atoms or of a mixture of such a higher carboxylic acid having up to 30 Mol % (anionic equivalents) of medium carboxylic acids and that it contains an amount of the amino group-containing monomer within the range of 30 to 80 wt % (based on the weight of the copolymer) and is soluble in water in the form of the salt.

2. A skin adhesive according to claim 1, characterised in that the copolymer contains an alkylester of acrylic and/or methacrylic acid having 4 to 14 carbon atoms in the alkyl group.

3. A skin adhesive according to claim 1 or 2, characterised in that it contains 2 to 200 wt % of a plasticiser for the salt of the copolymer.

4. A skin adhesive according to one or more of claims 1 to 3, characterised in that it contains 1 to 10 wt % of water based on the anhydrous copolymer.

5. An aqueous solution containing a skin adhesive, according to one or more of claims 1 to 4, and 20 to 90 wt % of water.

6. Use of the aqueous solution of the skin adhesive according to claim 5 for application onto a flat flexible substrate and subsequent drying.

7. A wound plaster containing a skin adhesive according to one or more of claims 1 to 4.

8. A transdermally effective skin-adhering pharmaceutical preparation containing a topical or systemic pharmaceutically active substance and a skin adhesive according to one or more of claims 1 to 4.

## Revendications

1. Adhésif à la peau, sensible à la pression et durablement élastique, contenant un sel d'un copolymère non réticulé, composé d'un monomère à mono-insaturation éthylénique contenant des groupements amino, susceptible de polymérisation radicalaire, et d'au moins un ester alkylique de l'acide acrylique et/ou méthacrylique,
caractérisé en ce que
le copolymère est le sel d'au moins un acide organique carboxylique supérieur à 8-20 atomes de carbone ou d'un mélange d'un tel acide carboxylique supérieur avec 30% en moles au maximum (des équivalents anioniques) d'acides carboxyliques moyens, il contient le monomère contenant des groupements amino dans une proportion de l'ordre de 30 à 80% en poids (par rapport au poids du copolymère) et il est soluble dans l'eau sous la forme de sel.

2. Adhésif a la peau selon la revendication 1, caractérisé en ce que le copolymère contient un ester alkylique de l'acide acrylique et/ou méthacrylique à 4-14 atomes de carbone dans le reste alkyle.

6

3. Adhésif à la peau selon la revendication 1 ou 2, caractérisé en ce qu'il contient de 2 à 200% en poids d'un plastifiant pour le sel du copolymère.

4. Adhésif à la peau selon l'une quelconque des revendications 1 à 3, caractérisé en ce qu'il contient de 1 à 10% en poids d'eau, par rapport au copolymère anhydre.

5. Solution aqueuse, contenant un adhésif à la peau selon l'une quelconque des revendications 1 à 4 et 20 à 90% en poids d'eau.

6. Utilisation de la solution aqueuse de l'adhésif à la peau selon la revendication 5, par application sur un substrat souple en nappe, suivie de séchage.

7. Sparadrap, contenant un adhésif à la peau selon l'une quelconque des revendications 1 à 4.

8. Médicament à action transdermique, adhérant à la peau, contenant une substance pharmaceutique à activité topique ou systémique et un adhésif à la peau salon l'une quelconque des revendications 1 à 4.